(19) **Europäisches Patentamt**

**European Patent Office**

**Office européen des brevets**

(11) **EP 1 107 952 B1**

(12) **EUROPEAN PATENT SPECIFICATION**

(45) Date of publication and mention
of the grant of the patent:
**29.01.2003 Bulletin 2003/05**

(51) Int Cl.[7]: **C07C 407/00**, C07C 409/14,
C07C 45/53, C07C 49/403,
C07C 29/132, C07C 35/08

(21) Application number: **99942457.5**

(22) Date of filing: **25.08.1999**

(86) International application number:
**PCT/US99/19311**

(87) International publication number:
**WO 00/012470 (09.03.2000 Gazette 2000/10)**

(54) **HYDROPEROXIDE DECOMPOSITION PROCESS**

HYDROPEROXIDZERSETZUNGSVERFAHREN

DECOMPOSITION DES HYDROPEROXYDES

(84) Designated Contracting States:
**BE DE GB IT NL**

(30) Priority: **26.08.1998 US 97982 P**

(43) Date of publication of application:
**20.06.2001 Bulletin 2001/25**

(73) Proprietor: **E.I. DUPONT DE NEMOURS AND
COMPANY
Wilmington, DE 19808 (US)**

(72) Inventors:
• **DRULINER, Joe, Douglas
Newark, DE 19711 (US)**

• **KOB, Nicholas, Edward, III
Port Arthur, TX 77642 (US)**
• **LANE, Samuel, Livingston
Beaumont, TX 77708 (US)**
• **STOWE, Gerald, Thomas
Victoria, TX 77904 (US)**

(74) Representative: **Jones, Alan John
CARPMAELS & RANSFORD
43 Bloomsbury Square
London, WC1A 2RA (GB)**

(56) References cited:
**EP-A- 0 404 417       WO-A-98/34894**
**US-A- 4 238 415       US-A- 5 550 301**

**Description**

The invention generally relates to an improved catalytic process for decomposing alkyl or aromatic hydroperoxides to form a mixture containing the corresponding alcohol and ketone. In particular, the invention relates to decomposing a hydroperoxide by washing the feed with water to remove acid impurities, removing the majority of the water from the product stream, then contacting it with a catalytic amount of a heterogeneous catalyst.

BACKGROUND OF THE INVENTION

Industrial processes for the production of mixtures of cyclohexanol and cyclohexanone from cyclohexane are currently of considerable commercial significance and are well described in the patent literature. In accordance with typical industrial practice, cyclohexane is oxidized to form a reaction mixture containing cyclohexyl hydroperoxide (CHHP). The resulting CHHP is either decomposed or hydrogenated, optionally in the presence of a catalyst, to form a reaction mixture containing cyclohexanol and cyclohexanone. In the industry, such a mixture is known as a K/A (ketone/alcohol) mixture, and can be readily oxidized to produce adipic acid or caprolactam, which are important reactants in processes for preparing certain condensation polymers, notably polyamides. A high K/A ratio in the reaction mixture is generally preferred. Due to the large volumes of adipic acid consumed in these and other processes, improvements in processes for producing adipic acid and its precursors can be used to provide beneficial cost advantages.

Druliner et al., (WO 98/34894) used a heterogeneous gold catalyst in an improved catalytic process for decomposing alkyl or aromatic hydroperoxides to form a mixture containing the corresponding alcohol and ketone.

Two common problems in CHHP processes, especially in heterogeneous catalytic processes, are the presence of water and acidic byproducts in the CHHP-containing reaction mixture. Both of these can deactivate the catalysts, resulting in lower conversion rates and/or lower K/A ratios. One method to eliminate the acidic byproducts is by the addition of a neutralization agent, such as that described in U.S. Patent No.4,238,415. This, however, results in undesirable salts, which need to be removed from the final product. In situ drying of the reaction mixture to remove water has been used in both hydrogenation and decomposition processes (U.S. Patent Nos. 5,550,301 and 3,927,108), but these methods do not remove the acidic by-products along with the water.

Applicants have discovered that good conversion rates and good K/A ratios can be achieved by the addition of a set amount of water to the reaction mixture, and subsequent removal of the water before the heterogeneous decomposition step removes the acidic impurities.

Further improvements and options are needed for hydroperoxide decomposition to K/A mixtures in order to overcome the deficiencies inherent in the prior art. Other objects and advantages of the present invention will become apparent to those skilled in the art upon reference to the detailed description which hereinafter follows.

SUMMARY OF THE INVENTION

An improved process is provided in which a hydroperoxide is decomposed to form a mixture containing a corresponding alcohol and ketone. The improvement comprises the steps of a) adding water in the amount of 0.5-20% to a hydroperoxide containing mixture; b) removing the bulk of said water by a means such that water-soluble impurities are removed along with the water; c) removing the remaining water by a means such that not more than 2% of water remains in the reaction mixture; and d) decomposing said hydroperoxide by contacting the reaction mixture with a catalytic amount of a heterogeneous catalyst. The heterogeneous catalyst comprises Au (gold). The inventive process may optionally be performed using Au in the presence of other metals, preferably Pd. The catalysts may further, optionally, be supported on a suitable support member, such as $SiO_2$, $Al_2O_3$, carbon, zirconia, MgO or $TiO_2$.

The hydroperoxide is preferably cyclohexylhydroperoxide, and the decomposition reaction mixture is preferably the result of an oxidation reaction of cyclohexane.

The preferred means to remove the water in step b) is decanting, and the preferred means to remove the water in step c) is flashing.

DETAILED DESCRIPTION OF THE PREFERRED EMBODIMENTS

The present invention provides an improved process for conducting a hydroperoxide decomposition step in an industrial process in which an alkyl or aromatic compound is oxidized to form a mixture of the corresponding alcohol and ketone. In particular, cyclohexane can be oxidized to form a mixture containing cyclohexanol (A) and cyclohexanone (K). The industrial process involves two steps: first, cyclohexane is oxidized, forming a reaction mixture containing CHHP; second, CHHP is decomposed, forming a mixture containing K and A. As previously mentioned, processes for the oxidation of cyclohexane are well known in the literature and available to those skilled in the art.

The improved process can also be used for the decomposition of other alkane or aromatic hydroperoxides, for example, t-butyl hydroperoxide, cyclododecylhydroperoxide and cumene hydroperoxide.

Advantages of the present heterogeneous catalytic process, relative to processes employing homogenous metal catalysts, such as metal salts or metal/ligand mixtures, include longer catalyst life, improved yields of useful products, and the absence of soluble metal compounds. However, the use of heterogeneous catalysts subject the process to fouling by water and organic impurities from the oxidation reaction, especially acidic impurities.

Applicants have discovered a multi-step process that overcomes these obstacles:

a) adding water in the amount of 0.5-20% to a hydroperoxide containing mixture;

b) removing the bulk of said water by any means such that water-soluble impurities are removed along with the water,

c) removing the remaining water by any means such that not more than 2% of water remains in the reaction mixture; and

d) decomposing said hydroperoxide by contacting the reaction mixture with a catalytic amount of a heterogeneous catalyst.

The first two steps are washing by addition of a certain amount of water to the hydroperoxide-containing mixture, and subsequent removal of the bulk of the water by any means that will remove impurities from the reaction mixture along with the water, notably acidic by-products from the oxidation process such as organic mono- and di-basic acids. Water is added in the amount of 0.5-20% by weight of the hydroperoxide-containing mixture. Preferred is about 4%.

Removal of these impurities prevents fouling of the catalyst, which lowers the K/A ratio. A high K/A ratio is generally preferred. Methods to remove the water that will also remove impurities from the reaction mixture along with the water include, but are not limited to, decantation and extraction. A preferred method is decantation. The water is preferably removed to levels of less than about 5%.

The hydroperoxide is preferably cyclohexylhydroperoxide, and the hydroperoxide-containing mixture is preferably the result of an oxidation reaction of cyclohexane. Any oxidation process can be used, but one typical of that used in industry is that described in Druliner et al., U.S. Patent No. 4,326,084.

Next, the remaining water is removed in any manner, down to levels of less than 2%, preferably less than 0.5%, to eliminate deactivation of the catalyst by the water, which would lower the conversion rate. Methods to accomplish this include, but are not limited to, flashing, distillation and contact with drying agents. A preferred method is flashing.

The process can be either continuous or batch.

After removal of the remaining water, the dried decomposition reaction mixture containing the hydroperoxide is then contacted with a catalytic amount of a heterogeneous catalyst. This decomposition process can be performed under a wide variety of conditions and in a wide variety of solvents, including the initial alkane that is oxidized to the hydroperoxide. Since CHHP is typically produced industrially as a solution in cyclohexane from catalytic oxidation of cyclohexane, a convenient and preferred solvent for the decomposition process of the invention is cyclohexane. Such a mixture can be used as received from the first step of the cyclohexane oxidation process or after some of the constituents have been removed by known processes such as distillation or aqueous extraction to remove carboxylic acids and other impurities.

The preferred concentration of CHHP in the CHHP reaction mixture can range from 0.5% by weight to 100% (i.e., neat). In the industrially practiced route, the preferred range is from 0.5% to 3% by weight.

Suitable reaction temperatures for the process of the invention range from 80°C to 170°C. Temperatures from 110°C to 160°C are typically preferred. Reaction pressures can preferably range from 69 kPa to 2760 kPa (10-400 psi) pressure, and pressures from 276 kPa to 1380 kPa (40-200 psi) are more preferred. Reaction time varies in inverse relation to reaction temperature, and typically ranges from 1 to 30 minutes.

As noted previously, the heterogeneous catalysts of the invention comprise Au and Au compounds, preferably applied to suitable solid supports. The inventive process may also be performed using Au in the presence of other metals, preferably Pd. The metal to support percentage can vary from 0.01 to 50 percent by weight, and is preferably 0.1 to 10 wt. percent. Suitable and presently preferred supports include $SiO_2$ (silica), $Al_2O_3$ (alumina), C (carbon), $TiO_2$ (titania), MgO (magnesia) or $ZrO_2$ (zirconia). Zirconia is a particularly preferred support, and Au supported on alumina is a particularly preferred catalyst of the invention. A preferred catalyst is 0.1-10% Au/0.05-2%Pd on $\alpha$-alumina, more preferably 1%Au/0.1% Pd on $\alpha$-alumina.

Some of the heterogeneous catalysts can be obtained already prepared from manufacturers, or they can be prepared from suitable starting materials using methods known in the art. Supported gold catalysts can be prepared by any standard procedure known to give well-dispersed gold, such as sol-gel techniques, evaporative techniques or coatings from colloidal dispersions.

In particular, ultra-fine particle sized gold is preferred. Such small particulate gold (often smaller than 10 nm) can be prepared according to Haruta, M., "Size-and Support-Dependency in the Catalysis of Gold", Catalysis Today 36 (1997) 153-166 and Tsubota et al., Preparation of Catalysts V, pp. 695-704 (1991). Such gold preparations produce samples that are purple-pink in color instead of the typical bronze color associated with gold and result in highly dispersed gold catalysts when placed on a suitable support member. These highly dispersed gold particles typically are from 3 nm to 15 nm in diameter.

The catalyst solid support, including $SiO_2$, $Al_2O_3$, carbon, MgO, zirconia, or $TiO_2$, can be amorphous or crystalline, or a mixture of amorphous and crystalline forms. Selection of an optimal average particle size for the catalyst supports will depend upon such process parameters as reactor residence time and desired reactor flow rates. Generally, the average particle size selected will vary from 0.005 mm to 5 mm. Catalysts having a surface area larger than 10 $m^2$/g are preferred since increased surface area of the catalyst has a direct correlation with increased decomposition rates in batch experiments. Supports having much larger surface areas can also be employed, but inherent brittleness of high-surface area catalysts, and attendant problems in maintaining an acceptable particle size distribution, will establish a practical upper limit upon catalyst support surface area.

A "sol-gel technique" is a process wherein a free flowing fluid solution, "sol", is first prepared by dissolving suitable precursor materials such as colloids, alkoxides or metal salts in a solvent. The "sol" is then dosed with a reagent to initiate reactive polymerization of the precursor. A typical example is tetraethoxyorthosilicate (TEOS) dissolved in ethanol. Water, with trace acid or base as catalyst to initiate hydrolysis, is added. As polymerization and crosslinking proceeds, the free flowing "sol" increases in viscosity and can eventually set to a rigid "gel". The "gel" consists of a crosslinked network of the desired material, which encapsulates the original solvent within its open porous structure. The "gel" may then be dried, typically by either simple heating in a flow of dry air to produce a xerogel, or the entrapped solvent may be removed by displacement with a supercritical fluid such as liquid $CO_2$ to produce an aerogel. These aerogels and xerogels may be optionally calcined at elevated temperatures (>200°C) which results in products which typically have very porous structures and concomitly high surface areas.

In practice of the invention, the catalysts can be contacted with CHHP by formulation into a catalyst bed, which is arranged to provide intimate contact between catalysts and reactants. Alternatively, catalysts can be slurried with reaction mixtures using techniques known in the art. The process of the invention is suitable for batch or for continuous CHHP decomposition processes. These processes can be performed under a wide variety of conditions.

Adding air or a mixture of air and inert gases to CHHP decomposition mixtures provides higher conversions of process reactants to K and A, since some cyclohexane is oxidized directly to K and A, in addition to K and A being formed by CHHP decomposition. This ancillary process is known as "cyclohexane participation", and is described in detail in Druliner et al., U.S. Patent No. 4,326,084, the entire contents of which are incorporated by reference herein.

Moderate increases in conversion can be obtained by adding hydrogen to the reaction, which also results in the increase of the ratio of K to A produced.

A preferred process of the invention would comprise the steps of:

a) removing impurities in hydroperoxide mixture by adding 4% water;
b) removing said water to a level of less than 5% by decantation at 90 to 160°C and 1 to 300 psig (0.006 to 2.07 MPa gauge);
c) removing the remaining water to a level of less than about 0.5% by flashing at 70 to 250°C, and
d) decomposing the. cyclohexylhydroperoxide by contacting the reaction mixture with a catalytic amount of a Au catalyst, said catalyst comprising 1% Au and 0.1% Pd supported on a suitable supported on alumina.

The process of the present invention is further illustrated by the following non-limiting examples. In the examples, all temperatures are in degrees Celsius and all percentages are by weight unless otherwise indicated.

METHODS AND MATERIALS

The catalysts used in the following Examples were prepared as described in Druliner et al., WO 98/34894, the entire contents of which are incorporated by reference herein.

Abbreviations used hereafter are listed and defined below as follows:

CHHP:    Cyclohexylhydroperoxide
K:       Cyclohexanone
A:       Cyclohexanol

All percentages are expressed as weight percent, unless otherwise specified.

The catalyst used for all Examples was 10 g of I % Au/0.1% Pd supported on α-alumina. The reactor was a liquid full plug flow reactor, 30 inches (76 cm) with a ¼ inch (0.64 cm) diameter. Inlet and exit pressure was 150 psig (1.03 MPa gauge) controlled with a backpressure regulator. The feed consisted of 1.6% CHHP in cyclohexane, about 1% K and 2% A, and varying amounts of water and acid impurities. The acid impurities referred to in the Examples consisted of monobasic and dibasic acids which would be typical of those produced in cyclohexane oxidation such as adipic acid, succinic acid, formic acid, and hydroxycaproic acid, in approximately equal amounts. Analyses were performed on CHHP, K, and A by gas chromatography Cyclohexane, CHHP, K, and A were obtained from E. 1. du Pont de Nemours and Company, Wilmington, DE. The K/A ratio obtained after conversion of cyclohexylhydroperoxide over the catalyst was calculated using the equation:

$$\frac{(g \text{ K in product}) - (g \text{ K in feed})}{(g \text{ A in product}) - (g \text{ A in feed})}$$

To illustrate the dependence of water and acid impurities in the feed to the CHHP decomposition reaction, cyclohexylhydroperoxide-containing feed was fed over the catalyst with varying amounts of pure water, and water containing 10% acids. As shown in Examples 2,3, 5 and 6, the presence of water above 0.1% results in deactivation of the catalyst, giving lower CHHP conversion and a lower K/A ratio. As shown in Example 4, the presence of acid impurities, independent of the amount of water present, also results in deactivation of the catalyst, giving lower CHHP conversion and a lower K/A ratio.

EXAMPLE 1

10 g of the 1%Au-0.1%Pd catalyst was used to decompose a feed mixture containing 1,6% CHHP, 0.9% K, 1.9%A, 0.1% water, and 0.3% acids with the remainder of the mixture being cyclohexane. This mixture was fed over the catalyst at 8 ml/min at 150°C, and 150 psig (1.03 MPa gauge). The results are presented below in Table I:

Table I

| Time on Stream (hrs) | % CHHP conversion | K/A ratio |
| --- | --- | --- |
| 10 | 99.1% | 1.79 |
| 22 | 99.4% | 1.95 |
| 52 | 99.2% | 2.05 |
| 64 | 99.5% | 2.09 |

EXAMPLE 2

This Example illustrates the effect of pure water. Increasing the water has only a small effect on conversion rates, but greatly lowers the K/A ratio. Catalyst and reaction conditions were the same as those described in Example 1. Added to the feed at varying levels was pure water. The results are presented below in Table II:

Table II

| Time on Stream (hr) | %CHHP conversion with 0.2% added water | K/A ratio with 0.2% added water | %CHHP conversion with 0.9% added water | K/A ratio with 0.9% added water |
| --- | --- | --- | --- | --- |
| 6 | 99.3 | 1.95 | 99.6 | 2.00 |
| 18 | 98.1 | 2.06 | 99.3 | 1.02 |
| 42 | 97.2 | 2.12 | 96.6 | 0.74 |

EXAMPLE 3

This Example illustrates the effect of water containing low amounts of acids. Increasing the water/acid mixture has a significant effect on conversion rates and K/A ratio. Catalyst and reaction conditions were the same as those described in Example 1. Added to the feed at varying levels was water containing 10% acids. The % acid indicates the percentage of acid in the added water, not the percentage in the total feed mixture. The results are presented below in Table III:

Table III

| Time on Stream (hr) | %CHHP conversion with 0.2% added water/acid mixture | K/A ratio with 0.2% added water/acid mixture | CHHP conversion with 0.9% added water/acid mixture | K/A ratio with 0.9% added water/acid mixture |
|---|---|---|---|---|
| 6 | 99.2 | 1.64 | 99.5 | 0.64 |
| 18 | 99.2 | 1.25 | 95.8 | 0.52 |
| 42 | 96.7 | 1.14 | 80.1 | 0.60 |

EXAMPLE 4

This Example illustrates the effect of water containing higher amounts of acids. The higher presence of acid greatly increased the effect of the added water on the conversion rate and K/A ratio. Catalyst and reaction conditions were the same as those described in Example 1. The feed in this example contains 1.9% CHHP, 0.5% K, 2.0% A, 0.1% water, and 1% acids, where the percentage indicates the amount of acid present in the total feed. The results are shown below in Table IV:

Table IV

| Time on Stream (hrs) | % CHHP conversion | K/A ratio |
|---|---|---|
| 4 | 90.3 | 1.04 |
| 17 | 90.9 | 0.82 |
| 29 | 86.8 | 0.83 |

EXAMPLE 5

The Example illustrates the effect of temperature on the CHHP decomposition reaction. Catalyst and reaction conditions were the same as those described in Example I except for the varying temperature. Added to the feed at 0.2% was water containing 10% acids. The results are shown in Table V, below:

Table V

| Temperature (°C) | % CHHP conversion | K/A ratio |
|---|---|---|
| 135 | 89.0 | 1.13 |
| 160 | 99.5 | 1.21 |

EXAMPLE 6

The Example illustrates the effect of pressure on the CHHP decomposition reaction. Catalyst and reaction conditions were the same as those described in Example 1 except for the varying pressure. Added to the feed at 0.2% was water containing 10% acids. The results are shown in Table VI, below:

Table VI

| Time on Stream (hr) | %CHHP conversion at 115 psig (0.79 MPa gauge) | K/A ratio at 115 psig (MPa gauge) | CHHP conversion at 225 psig (1.55 MPa gauge) | K/A ratio at 225 psig (1.55 MPa gauge) |
|---|---|---|---|---|
| 24 | 98.9 | 2.05 | 99.0 | 1.23 |
| 38 | 97.0 | 1.76 | - | - |
| 42 | - | - | 97.0 | 1.09 |

**Claims**

1. An improved process for decomposing a hydroperoxide, to form a mixture containing a corresponding alcohol and ketone, the improvement comprising the steps of: a) adding water in the amount of 0.5-20% to a hydroperoxide-containing mixture; b) removing the bulk of said water by a means such that water-soluble impurities are removed along with the water; c) removing the remaining water by a means such that not more than 2% of water remains in the reaction mixture; and d) decomposing said hydroperoxide by contacting the reaction mixture with a catalytic amount of a heterogeneous catalyst, wherein the catalyst is comprised of gold.

2. The process according to Claim 1 wherein the heterogeneous catalyst is supported on a catalyst support member.

3. The process according to Claim 2 wherein the catalyst support member is selected from the group consisting of $SiO_2$, $Al_2O_3$, carbon, $TiO_2$, MgO, and zirconia.

4. The process according to Claim 1 wherein the hydroperoxide is cyclohexylhydroperoxide.

5. The process according to Claim 1 wherein the decomposition reaction temperature is from 80°C to 170°C, and decomposition reaction pressure is from 69 kPa to 2760 kPa.

6. The process according to Claim 5 wherein the reaction pressure is from 276 kPa to 1380 kPa.

7. The process according to Claim 1 wherein the reaction mixture contains from 0.5 to 100 percent by weight cyclohexyl hydroperoxide.

8. The process according to Claim 1 wherein the process is run in the presence of cyclohexane.

9. The process according to Claim 1 wherein the process is run in the presence of added oxygen.

10. The process according to Claim 3 wherein the gold is supported on zirconia.

11. The process according to Claim 10 wherein the gold is from 0.1 to 10 wt. percent of the catalyst and support member.

12. The process according to Claim 1 wherein a metal from 0.05 to 2% wt. percent of the catalyst and support member is also present with gold.

13. The process according to Claim 12 wherein the metal is Pd.

14. The process according to Claim 2 wherein the gold is present on the support member as well-dispersed particles having a diameter from 3 nm to 15 nm.

15. The process according to Claim 13 wherein the catalyst is 1.0 percent by weight Au with 0.1 percent by weight Pd supported on alumina.

16. The process according to Claim 1 wherein the gold catalyst is in the form of a sol-gel compound.

17. The process according to Claim 1 wherein the process is run in the presence of added hydrogen.

18. The process according to Claim 1 wherein the means to remove water in step b) is decanting.

19. The process according to Claim 18 wherein the means to remove water in step c) is flashing.

20. The process according to Claim 1 wherein the hydroperoxide-containing mixture. results from the oxidation of cyclohexane.

**Patentansprüche**

1. Verbessertes Verfahren zur Zersetzung eines Hydroperoxids, um ein Gemisch, enthaltend einen entsprechenden Alkohol und Keton, zu erzeugen, wobei die Verbesserung die Schritte umfaßt: a) Hinzufügen von Wasser in dem Anteil von 0,5-20% zu einem Hydroperoxid enthaltenden Gemisch; b) Entfernen der Masse des Wassers durch ein Mittel derart, daß wasserlösliche Verunreinigungen zusammen mit dem Wasser entfernt werden; c) Entfernen des restlichen Wassers durch ein Mittel, derart, daß nicht mehr als 2% Wasser in dem Reaktionsgemisch verbleiben; und d) Zersetzen des Hydroperoxids durch Inkontaktbringen des Reaktionsgemischs mit einer katalytischen Menge eines heterogenen Katalysators, wobei der Katalysator aus Gold besteht.

2. Verfahren nach Anspruch 1, wobei der heterogene Katalysator auf einem Katalysatorträgerbestandteil geträgert ist.

3. Verfahren nach Anspruch 2, wobei der Katalysatorträgerbestandteil aus der Gruppe, bestehend aus $SiO_2$, $Al_2O_3$, Kohlenstoff, $TiO_2$, MgO und Zirconiumdioxid, ausgewählt ist.

4. Verfahren nach Anspruch 1, wobei das Hydroperoxid Cyclohexylhydroperoxid ist.

5. Verfahren nach Anspruch 1, wobei die Reaktionstemperatur der Zersetzung von 80°C bis 170°C beträgt und der Reaktionsdruck der Zersetzung von 69 kPa bis 2760 kPa beträgt.

6. Verfahren nach Anspruch 5, wobei der Reaktionsdruck von 276 kPa bis 1380 kPa beträgt.

7. Verfahren nach Anspruch 1, wobei das Reaktionsgemisch von 0,5 bis 100 Gewichtsprozent Cyclohexylhydroperoxid enthält.

8. Verfahren nach Anspruch 1, wobei das Verfahren in Anwesenheit von Cyclohexan durchgeführt wird.

9. Verfahren nach Anspruch 1, wobei das Verfahren in Anwesenheit von hinzugefügtem Sauerstoff durchgeführt wird.

10. Verfahren nach Anspruch 3, wobei das Gold auf Zirconiumdioxid geträgert ist.

11. Verfahren nach Anspruch 10, wobei das Gold von 0,1 bis 10 Gewichtsprozent des Katalysator- und Trägerbestandteils ausmacht.

12. Verfahren nach Anspruch 1, wobei ein Metall mit 0,05 bis 2 Gew.-% von dem Katalysator- und Trägerbestandteil ebenfalls mit Gold vorhanden ist.

13. Verfahren nach Anspruch 12, wobei das Metall Pd ist.

14. Verfahren nach Anspruch 2, wobei das Gold auf dem Trägerbestandteil als gut dispergierte Teilchen mit einem Durchmesser von 3 nm bis 15 nm vorhanden ist.

15. Verfahren nach Anspruch 13, wobei der Katalysator 1,0 Gewichtsprozent Au mit 0,1 Gewichtsprozent Pd, geträgert auf Aluminiumoxid, sind.

16. Verfahren nach Anspruch 1, wobei der Goldkatalysator in der Form einer Sol-Gel-Verbindung ist.

17. Verfahren nach Anspruch 1, wobei das Verfahren in Anwesenheit von hinzugefügtem Wasserstoff durchgeführt wird.

18. Verfahren nach Anspruch 1, wobei das Mittel zur Entfernung von Wasser in Schritt b) Dekantieren ist.

19. Verfahren nach Anspruch 18, wobei das Mittel zur Entfernung von Wasser in Schritt c) schnelles Verdampfen ist.

20. Verfahren nach Anspruch 1, wobei das Hydroperoxid enthaltende Gemisch aus der Oxidation von Cyclohexan entsteht.

**Revendications**

1. Procédé amélioré pour la décomposition d'un hydroperoxyde, pour former un mélange contenant un alcool et une cétone correspondants, l'amélioration comprenant les étapes: a) d'ajout d'eau dans la quantité de 0,5-20% à un mélange contenant l'hydroperoxyde; b) de retrait de la majeure partie de ladite eau par un moyen tel que les impuretés solubles dans l'eau sont retirées avec l'eau; c) de retrait de l'eau restante par un moyen tel qu'il ne reste pas plus de 2% d'eau dans le mélange réactionnel; et d) de décomposition dudit peroxyde par une mise en contact du mélange réactionnel avec une quantité catalytique d'un catalyseur hétérogène, dans lequel le catalyseur comprend de l'or.

2. Procédé suivant la revendication 1, dans lequel le catalyseur hétérogène est supporté sur un élément support de catalyseur.

3. Procédé suivant la revendication 2, dans lequel l'élément support de catalyseur est choisi dans le groupe constitué de $SiO_2$, $Al_2O_3$, de carbone, de $TiO_2$, de MgO et de zircone.

4. Procédé suivant la revendication 1, dans lequel l'hydroperoxyde est le cyclohexylhydroperoxyde.

5. Procédé suivant la revendication 1, dans lequel la température de 1a réaction de décomposition est de 80°C à 170°C et la pression de la réaction de décomposition est de 69 kPa à 2760 kPa.

6. Procédé suivant la revendication 5, dans lequel la pression de la réaction est de 276 kPa à 1380 kPa.

7. Procédé suivant la revendication 1, dans lequel le mélange réactionnel contient de 0,5 à 100 pour-cent en poids de cyclohexylhydroperoxyde.

8. Procédé suivant la revendication 1, dans lequel le procédé est mené en présence de cyclohexane.

9. Procédé suivant la revendication 1, dans lequel le procédé est mené en présence d'un ajout oxygène.

10. Procédé suivant la revendication 3, dans lequel l'or est supporté sur de la zircone.

11. Procédé suivant la revendication 10, dans lequel dans lequel l'or constitue de 0,1 à 10 pour-cent en poids du catalyseur et de l'élément support.

12. Procédé suivant la revendication 1, dans lequel un métal de 0,05 à 2% pour-cent en poids du catalyseur et de l'élément support est également présent avec l'or.

13. Procédé suivant la revendication 12, dans lequel le métal est Pd.

14. Procédé suivant la revendication 2, dans lequel l'or est présent sur l'élément support sous forme de particules bien dispersées possédant un diamètre de 3 nm à 15 nm.

15. Procédé suivant la revendication 13, dans lequel le catalyseur correspond à 1,0 pour-cent en poids de Au avec 0,1 pour-cent en poids de Pd supportés sur de l'alumine.

16. Procédé suivant la revendication 1, dans lequel le catalyseur d'or est sous la forme d'un composé sol-gel.

17. Procédé suivant la revendication 1, dans lequel le procédé est mené en présence d'un ajout d'hydrogène.

18. Procédé suivant la revendication 1, dans lequel le moyen pour retirer l'eau dans l'étape b) est une décantation.

19. Procédé suivant la revendication 18, dans lequel le moyen pour retirer l'eau dans l'étape c) est une vaporisation instantanée.

20. Procédé suivant la revendication 1, dans lequel le mélange contenant l'hydroperoxyde résulte de l'oxydation de cyclohexane.